# EUROPÄISCHE PATENTSCHRIFT

(11) **EP 3 943 125 B1**
(45) Veröffentlichungstag und Bekanntmachung des Hinweises auf die Patenterteilung: **12.04.2023**
(21) Anmeldenummer: 21177423.7
(22) Anmeldetag: 02.06.2021
(51) Int. Cl.: A61L 9/20, F24F 8/22, F24F 8/80, B60H 1/24, B60H 3/00, F24F 8/108, F24F 11/30, F24F 110/50, F24F 110/64

(54) **VORRICHTUNG ZUR DESINFEKTION VON LUFT IN INNENRÄUMEN**
DEVICE FOR DISINFECTING AIR IN INTERIORS
DISPOSITIF DE DÉSINFECTION DE L'AIR DANS DES ESPACES INTÉRIEURS

(30) Priorität: 21.07.2020 DE 102020119225
(43) Veröffentlichungstag der Anmeldung: 26.01.2022
(73) Patentinhaber: Landers, Henry, 13357 Berlin (DE)
(72) Erfinder: Landers, Henry, 13357 Berlin (DE)
(74) Vertreter: Zimmermann & Partner Patentanwälte mbB

(56) Entgegenhaltungen:
- WO-A1-2005/028965
- KR-A- 20060 004 541
- US-A1- 2005 163 652
- US-A1- 2018 021 468
- US-B2- 10 046 266

## Beschreibung

Die vorliegende Erfindung betrifft eine Vorrichtung zur Desinfektion von Luft in Innenräumen, insbesondere von Luft in Innenräumen von Fahrzeugen zur Personenbeförderung und in Gebäuden.

Luftübertragbare Keime (Erreger) wie Bakterien, zum Beispiel Tuberkulosebakterien und Viren, insbesondere Influenza- und Coronaviren stellen eine große Herausforderung für die Gesundheit der Bevölkerung und die wirtschaftliche Leistungsfähigkeit eines Landes, im Fall einer Pandemie gegebenenfalls sogar weltweit dar. Allein in Deutschland können pro Jahr 11.000 bis 35.000 Menschen an den Folgen einer Infektion mit Influenzavieren- oder Coronaviren sterben. Allein am Coronavirus SARS-CoV-2 starben in Deutschland laut Angaben des Robert Koch-Instituts von Januar 2020 bis April 2021 ca. 80.000 Menschen.

Zur Reduktion oder sogar Verhinderung der Ausbreitung von derartigen Krankheitserregern ist es erstrebenswert, deren Konzentration insbesondere in geschlossenen Räumen möglichst gering zu halten. Dies betrifft insbesondere den Personentransport im Nah- und Fernverkehr (Züge, U-Bahn, S-Bahn, Busse, Großraumtaxis und Straßenbahn, aber auch Flugzeuge) sowie Räume in Schulen, Kindergärten, Arztpraxen, Behandlungs- und Warteräume, und Räume in öffentlichen Gebäuden sowie in Bürogebäuden. Hierzu ist anzumerken, dass in Deutschland im Jahr 2019 ca. 2,6 Milliarden Mal die Deutsche Bundesbahn für Fahrten genutzt wurde. Allein in Berlin waren es zur gleichen Zeit 3,5 Millionen Fahrten mit öffentlichen Verkehrsmitteln pro Tag. Diese Zahlen verdeutlichen, wie dicht die Verkehrsnetze sind. Dementsprechend können sich Luftübertragbare Krankheitserreger schnell (ggf. sogar weltweit/pandemisch) verbreiten.

Ultraviolett-Luftdesinfektionsvorrichtung zur Desinfektion von Raumluft sind an sich bekannt. Sie sind unter anderem aus Geräusch-, Komfort-, Platz- und/oder Kostengründen jedoch vergleichsweise wenig verbreitet, insbesondere im öffentlichen Personennahverkehr aber auch in Bussen und Bahnen im Fernverkehr praktisch nicht vorhanden. Eine Nachrüstung mit entsprechenden Vorrichtungen ist aus diesem Gründen bisher häufig nicht attraktiv oder sogar nicht sinnvoll möglich.

So offenbart die DE 20 2013 000 809 U1 eine Ultraviolett-Luftdesinfektionsvorrichtung zur Desinfektion von Raumluft, welche neben einer Desinfektionseinheit mit einem UV-Strahler und einer aktiven Luftführungseinrichtung eine Beleuchtungseinheit mit einem Leuchtmittel zum Emittieren von Licht im sichtbaren Wellenlängenbereich aufweist. Diese UV-Luftdesinfektionsvorrichtung kann auch als Hängevorrichtung zur Anbringung an einer Raumdecke ausgebildet sein. Sie eignet sich insbesondere zur Verwendung in landwirtschaftlichen Einrichtungen, wie beispielsweise Ställen oder zur Verwendung in medizinischen Einrichtungen, insbesondere in Krankenhäusern und Operationssälen. Aufgrund des Aufbaus dieser Luftdesinfektionsvorrichtung bzw. der Luftführung/-Erzeugung durch diese Luftdesinfektionsvorrichtung eignet sie sich insbesondere weniger für Fahrgastinnenräume, da dieser Aufbau bei der beschränkten Bauhöhe und dem wünschenswerten Luftdurchsatz im Betrieb zu einer den Komfort einschränkenden Geräuschentwicklung neigt. Dadurch können hohe Luftströmungen entstehen, die die zulässigen Normen, die für öffentliche Verkehrsmittel gelten, überschreiten. Außerdem können unerwünschte Schwingungen des Gesamtsystems während einer Fahrt auftreten. Zudem kann ein nicht wünschenswertes Austreten von möglicherweise Passagiere bzw. Besucher schädigendem UV-Licht aus dieser Ultraviolett-Luftdesinfektionsvorrichtung nicht sicher ausgeschlossen werden. Zudem kann es zu Verschmutzungen der Ultraviolett-Luftdesinfektionsvorrichtung im Langzeitbetrieb kommen.

Weitere herkömmliche UV-Luftdesinfektionsvorrichtungen mit einfacher ein- oder ausgangsseitiger Ansaugung werden in den Dokumenten US 2005/163652 A1, KR 2006 0004541 A und US 2018/021468 A1 beschrieben. Zudem sind aus WO 2005/028965 A1 und US 10 046 266 B2 Desinfektionssysteme mit seriell angeordneten Lüftern bekannt.

Im Hinblick auf das oben Gesagte, schlägt die vorliegende Erfindung eine Vorrichtung gemäß Anspruch 1 vor.

Gemäß einem Ausführungsbeispiel weist eine Vorrichtung zur Desinfektion von Luft in Innenräumen, im Folgenden auch als Innenraumluftdesinfektionsvorrichtung bezeichnet, Folgendes auf Ein Gehäuse mit einer Längserstreckung und einer Oberseite zum Montieren an einer Innenwand des Innenraums, insbesondere einer Innendecke des Innenraums, einen Lufteinlass an einer der Oberseite gegenüberliegenden Unterseite des Gehäuses, sodass die Luft von unten durch den Lufteinlass quer zur Längserstreckung des Gehäuses angesaugt werden kann, wobei der Lufteinlass eine erste Querschnittsfläche aufweist, einen Luftauslass an einer die Oberseite mit der Unterseite verbindende Stirnseite des Gehäuses, sodass die Luft durch den Luftauslass zumindest im Wesentlichen in Längserstreckung des Gehäuses in den Innenraum abgegeben werden kann, wobei der Luftauslass eine zweite Querschnittsfläche aufweist, die kleiner als die erste Querschnittsfläche ist, einen im Gehäuse vom Lufteinlass zum Luftauslass verlaufenden Strömungskanal, eine im Gehäuse angeordnete und/oder einen Teil des Gehäuses bildende Desinfektionszelle, die einen Teil des Strömungskanals bildet und in der der Strömungskanal einen Strömungsquerschnitt aufweist, der kleiner als die zweite Querschnittsfläche ist, wobei die Desinfektionszelle eingangsseitig mindestens einen ersten Lüfter zum Ansaugen von Luft vom Lufteinlass und Abgeben der angesaugten Luft in die Desinfektionszelle und ausgangsseitig mindestens einen zweiten Lüfter zum Ansaugen der Luft aus der Desinfektionszelle und Abgeben der angesaugten Luft zum Luftauslass aufweist, und wobei der erste Lüfter und der zweite Lüfter jeweils mindestens einen oder mehrere Rotorflügel aufweisen, der/die so ausgeführt ist/sind, dass er/sie in Strömungsrichtung der Luft gesehen keinen direkten Durchtritt von Licht gestattet/gestatten, und mindestens eine in der Desinfektionszelle im Strömungskanal angeordnete UV-Lichtquelle zum Bestrahlen der durch den Strömungskanal strömenden Luft.

Die Formulierung, dass die Luft durch den Luftauslass zumindest im Wesentlichen in Längserstreckung des Gehäuses in den Innenraum abgegeben werden kann, bedeutet, dass eine Abweichung der mittleren Strömungsrichtung am Luftauslass von der Längserstreckung und/oder der mittleren Strömungsrichtung im Strömungskanal in der Desinfektionszelle um höchstens 25°, 15° oder sogar nur höchstens 10° oder 5° abweicht.

Diese Innenraumluftdesinfektionsvorrichtung ermöglicht einen äußerst geräuscharmen Betrieb bei hohem Luftdurchsatz in kompakter Bauweise. Aufgrund der gewählten Verhältnisse der Querschnitte des Strömungskanals am Lufteinlass, Luftauslass und in der Desinfektionszelle sowie deren Orientierung zueinander kann die Luft effektiv aus dem Innenraum angesaugt, durch die Vorrichtung geführt und UV-Licht-behandelt effektiv wieder in den Innenraum abgegeben werden, ohne dass dabei UV-Licht in den Innenraum abgegeben wird.

So können Mikroorganismen in der Luft bei einer exemplarischen Größe der Innenraumluftdesinfektionsvorrichtung von 128 cm x 39 cm x 20 cm, einem vergleichsweise geringen Gewicht von etwa 12 kg, einer elektrischen Gesamtleistung im Durchschnitt von 200 W inklusive der exemplarisch als Quecksilberniederdrucklampen ausgeführten UV-Lichtquellen, mit einem Wellenlängenspektrum, sodass bei Bestrahlung der Luft praktisch kein Ozon erzeugt wird (Wellenlängen >240 nm, insbesondere >=250 nm oder sogar >=300 nm), und Pumpraten von bis zu 120 m³/h zu 99,97 % unschädlich gemacht werden, ohne dabei unerwünschte Nebenprodukte wie Ozon und UV-Licht in den Innenraum abzugeben.

Diese Ausführungsform eignet sich besonders für Passagierinnenräume von Bussen und Bahnen. Da lediglich ein elektrischer Anschluss geringer Leistung (je nach Ausführung von bspw. 100 W bis 300 W) zur Versorgung der hierin beschriebenen Innenraumluftdesinfektionsvorrichtung erforderlich ist, können die Innenräume bestehender Fahrzeuge (Verkehrssysteme) und Gebäude (z.B. Schulen und Kindergärten) auch vergleichsweise einfach und platzsparend nachgerüstet werden.

Die hierin beschriebenen Innenraumluftdesinfektionsvorrichtungen ermöglichen somit:
eine vergleichsweise kostengünstige ganzjährige Prävention von Masseninfektionen verursacht durch Viren, Bakterien, Hefen und Schimmelpilze, die in der Luft in der Öffentlichkeit verbreitet werden können,
eine Verbesserung der Lebensqualität von Passagieren, Kindern bzw. Besuchern von öffentlichen und privaten Räumen,
eine signifikante Kosteneinsparungen im Gesundheitswesen,
eine Reduzierung der Krankentage von Mitarbeitern / Personal und damit verbundener zusätzlicher Kosten,
eine effektive Vorbeugung und Unterstützung im Falle einer Pandemie,
eine Reduzierung der CO2-Emissionen durch eine erhöhte Attraktivität der öffentlichen Verkehrsmittel, sowie
eine Verbesserung der nationalen Sicherheit im Falle einer Bedrohung / eines Terroranschlags mit biologischen B- oder C-Waffen in öffentliche Verkehrssystemen.

Um eine möglichst hohe Desinfektionsrate zu erreichen kann vorgesehen sein, dass die Desinfektionszelle UV-Licht reflektierende innere Oberfläche(n) aufweist, die den Strömungskanal zumindest teilweise begrenzt.

Dazu kann zudem vorgesehen sein, dass die mindestens eine UV-Lichtquelle beabstandet vom Rand des Strömungskanals angeordnet (und in Betrieb somit von der Luftströmung umgeben) ist.

Außerdem sind typischerweise mehrere UV-Lichtquellen zum Bestrahlen der durch den Strömungskanal strömenden Luft vorgesehen sind, die auch unterschiedliche Spektraleigenschaften aufweisen können.

Alternativ zu Niederdrucklampen können auch entsprechende Leistung LEDs bzw. LED-Arrays als UV-Lichtquellen verwendet werden. Dadurch kann der Energieverbrauch gegebenenfalls weiter gesenkt werden.

Es hat sich herausgestellt, dass der Strömungskanal besonders effektiv und geräuscharm mit zu desinfizierender Luft durchströmt werden kann, wenn eine Flächennormale der ersten Querschnittsfläche im Wesentlichen senkrecht zum Strömungsquerschnitt des Strömungskanals in der Desinfektionszelle ist, d.h. eine Umlenkung der typischerweise großflächig am Lufteinlass angesaugten Luft um ca. 90° zur Desinfektionszelle hin erfolgt.

Wegen unterschiedlicher Gegebenheiten in Verkehrsmitteln kann es für spezielle Lösungen aber auch sinnvoll sein, von dieser Ausführungsform abzuweichen und eine Luftumlenkung um ca. 180° vorzusehen. Z.B. kann dies der Fall günstig sein, wenn ein Verkehrsmittel mit bspw. geringer Deckenhöhe mit einem oder mehreren Innenraumluftdesinfektionsvorrichtungen ausgestattet werden soll. In diesem Fall kann der Lufteinlass (und die weiter unten beschriebene Steuereinheit) um 90 ° zur Unterseite hin geneigt sein, der Gehäuseteil mit dem Lufteinlass (und der Steuereinheit) an einer Decke und die Desinfektionszelle bzw. der mittlere und/oder der Luftausströmbereich der Innenraumluftdesinfektionsvorrichtung(en) an einer Seitenwand befestigbar sein.

Aus analogen Gründen wird der Strömungsquerschnitt am bzw. zum Luftauslass typischerweise wieder vergrößert, bleibt dabei typischerweise jedoch geringer als am Lufteinlass, und/oder eine Orientierung der ersten Querschnittsfläche des Lufteinlasses von einer Orientierung der zweiten Querschnittsfläche des Luftauslasses und/oder einer Orientierung des Strömungsquerschnitt des Strömungskanals in der Desinfektionszelle abweicht. Beispielsweise kann die zweite Querschnittsfläche um bis zu 20°, bis zu 30° oder sogar bis zu 45° gegenüber dem Strömungsquerschnitt des Strömungskanals in der Desinfektionszelle geneigt sein.

Der Lufteinlass kann durch einen Schutzgitter gebildet sein.

Das Schutzgitter (auch mit Metallgeflecht) kann einen mechanischen Schutz für den Strömungskanal gegen Beschädigung bereitstellen, und/oder als Filter mit einer Maschenweite von typischerweise mindestens 0,1 cm oder sogar mindestens 1 cm ausgeführt sein.

Typischerweise sind dem Schutzgitter in Strömungsrichtung Lamellen zum besonders geräuscharmen Umlenken der einströmenden Luft zur Desinfektionszelle nachgelagert. Die Lamellen dienen auch der gleichmäßig flächigen Luftansaugung und Luftausströmung.

Dazu kann vorgesehen sein, dass die Lamellen in einem Querschnitt entlang des Strömungskanals einem jeweiligen glatten, gekrümmten Kurvenabschnitt folgen.

Bevorzugt sind die Lamellen am Lufteinlass so ausgeführt bzw. angeordnet, dass zwischen den Lamellen jeweilige Strömungsbereiche mit einem Strömungswiderstand gebildet werden, und Strömungsbereiche, die näher am ersten Lüfter verlaufen / liegen, einen höheren Strömungswiderstand aufweisen als Strömungsbereiche, die weiter vom ersten Lüfter verlaufen / liegen.

Dies ermöglicht, dass die Luft am Lufteinlass gleichmäßig angesaugt werden kann.

Analog dazu kann auch der Luftauslass durch einen entsprechenden Schutzgitter gebildet sein, dem typischerweise in Strömungsrichtung Lamellen zum Führen der ausströmenden Luft nachgelagert sind.

Außerdem kann vorgesehen sein, dass im Wandbereich des Strömungskanals in der Desinfektionszelle Strömungsumlenkelemente angeordnet sind, die den Strömungskanal in einem Querschnitt entlang des Strömungskanals jeweils wellenförmig, asymmetrisch und/oder einer jeweiligen glatten Kurve folgend begrenzen.

Es hat sich herausgestellt, dass derartige Strömungsumlenkelemente zu einer geräuscharmen, leichten Verwirbelung der Luft im Bereich der UV-Lichtquellen führen, die eine besonders hohe Desinfektionsrate ermöglicht.

Insbesondere kann vorgesehen sein, dass in dem Querschnitt entlang des Strömungskanals mindestens ein Strömungsumlenkelement an einem ersten Abschnitt des Wandbereichs, insbesondere einem oberen Abschnitt des Wandbereichs angeordnet ist, und ein weiteres Strömungsumlenkelement an einem zum ersten Abschnitt des Wandbereichs gegenüberliegenden zweiten Abschnitt des Wandbereichs, insbesondere einem unteren Abschnitt des Wandbereichs und in Strömungsrichtung versetzt zu dem mindestens einem Strömungsumlenkelement angeordnet ist.

Die Desinfektionszelle weist typischerweise eine Stützstruktur zur Befestigung an einer Wand insbesondere einer Innendecke des Innenraums auf. Es ist prinzipiell aber auch möglich, die Oberseite der Luftdesinfektionsvorrichtung / deren Stützstruktur an einer Seitenwand des Innenraums anzubringen. Zumindest in Fahrzeugen erfolgt die Montage typischerweise aber an einer Innendecke des Innenraums.

Beispielsweise kann die Stützstruktur durch einen (stabilen und/oder starren) Metallrahmen, insbesondere einen Aluminiumrahmen gebildet werden bzw. diesen aufweisen.

Typischerweise sind die Lüfter an der Stützstruktur befestigt.

Außerdem kann vorgesehen sein, dass das Gehäuse von der Stützstruktur getragen wird.

Wenn die Befestigung der Luftdesinfektionsvorrichtung im Innenraum zumindest teilweise über deren Stützstruktur erfolgt, wirkt sich dies positiv auf die Stabilität des Gesamtsystems und das Schwingungsverhalten während des Betriebs bzw. der Fahrt eines damit ausgerüsteten Fahrzeugs aus.

Diese Ausführungsform der Desinfektionszelle wird im Folgenden auch als Sicherheitsdesinfektionszelle bezeichnet.

An der Stützstruktur der Sicherheitsdesinfektionszelle kann eine Trägerstruktur für die mindestens eine UV-Lichtquelle befestigt sein.

Alternativ dazu kann die Trägerstruktur separat zur Desinfektionszelle bzw. zur Stützstruktur für eine Befestigung an einer Wand, insbesondere einer Innendecke des Innenraums ausgelegt sein.

Eine derartige Trägerstruktur erleichtert das Auswechseln der mindestens einen UV-Lichtquelle.

Typischerweise bestehen die Trägerstruktur und die Stützstruktur aus dem gleichen Material, zum Beispiel Aluminium.

Dies wirkt sich ebenfalls positiv auf die Stabilität und das Schwingungsverhalten des Gesamtsystems aus.

Mindestens eine innere Oberfläche der Luftdesinfektionsvorrichtung, insbesondere eine innere Oberfläche der Desinfektionszelle kann antimikrobakteriell ausgeführt und/oder photokatalytisch aktiv sein bzw. mit eine photokatalytisch aktiven Schicht, insbesondere einer TiO₂ - haltigen Schicht mit einer typischen Schichtdicke im Bereich 5 nm - 1500 nm versehen sein.

Dadurch können die Oberflächen (gegebenenfalls auch außerhalb der Desinfektionszelle) selbstreinigend ausgeführt und/oder die luftreinigende und/oder luftdesinfizierende Wirkung in der Desinfektionszelle erhöht werden.

Durch die photokatalytische Wirkung des Titandioxids (oder einer anderen photokatalytisch aktiven Schicht) können sowohl Luftschadstoffe /-Verbindungen oxidativ abgebaut als auch Mikroorganismen zerstört werden. Zudem kann die Bildung von Kondenswassertropfen durch aufkommenden Niederschlag von Aerosolen an Elementen im inneren des Gerätes durch die Nanostruktur der photokatalytischen Beschichtung verhindert werden. Die Abtrocknung von entstehender Feuchtigkeit im Gerät wird dadurch im Normalbetrieb unterstützt da möglicherweise sich sammelnde Tropfen auf der photokatalytischen Oberfläche zu einem hauch dünnen leicht trocknenden Film verfließen.

Optional kann eine oder mehrere separate Lichtquellen zur Bestrahlung der photokatalytischen Oberfläche(n) außerhalb der (lichtdichten) Desinfektionszelle vorgesehen sein, die zum Beispiel während eines Reinigungszyklus aktiviert wird/werden.

Die separate Lichtquelle(s) kann eine Leuchtstofflampe, eine Plasmalampe, aber auch eine Tageslichtlampe oder eine LED sein, die aus Sicherheitsgründen beispielsweise eine (nennenswerte) Emission nur oberhalb von 240 nm, z.B. oberhalb von 250 oder sogar 350 nm aufweist/ aufweisen.

Um das Austreten von UV-Licht aus der Desinfektionszelle zu vermeiden, ist typischerweise vorgesehen, dass der erste Lüfter und der zweite Lüfter jeweils eine Rotorachse aufweisen und mindestens einen um die Rotorachse rotierbaren Rotorflügel aufweisen, wobei der mindestens eine Rotorflügel jeden parallel zur Rotorachse auftreffenden Lichtstrahl absorbieren und/oder reflektieren kann, und/oder wobei der jeweilige mindestens eine Rotorflügel ein Lichtlabyrinth bildet.

Der jeweilige mindestens eine Rotorflügel kann entlang einer Schraubenlinie um die Rotorachse geformt sein.

Eine jeweilige Flügelspitze des Rotorflügel, insbesondere des eingangsseitigen Lüfters kann jedoch von der Schraubenline abweichen.

Dies ermöglicht eine geräuscharme leichte Verwirbelung, die eingangsseitig zu einer verbesserten Desinfektionseffizienz beitragen kann.

Der erste Lüfter und der zweite Lüfter können typischerweise mit der gleichen Pumpleistung von beispielsweise jeweils maximal 25 W oder sogar nur jeweils maximal 10 W betrieben werden.

Außerdem können der erste und zweite Lüfter zumindest im Wesentlichen gleich aufgebaut sein.

Die Rotorachsen vom ersten und zweiten Lüfter können zumindest im Wesentlichen parallel orientiert sein.

Insbesondere bei besonders flachen Aufbauten, können die Achsen der ersten und zweiten Lüfter aber auch zueinander geneigt sein.

Außerdem kann vorgesehen sein, zwei oder noch mehr erste Lüfter, die in Strömungsrichtung nebeneinander angeordnet sind, und/oder zwei oder sogar noch mehr zweite Lüfter, die in Strömungsrichtung nebeneinander angeordnet sind, einzusetzen.

Dadurch lässt sich der Luftdurchsatz erhöhen.

Typischerweise wird die gleiche Anzahl von ersten und zweiten Lüftern verwendet.

Gemäß eines Ausführungsbeispiels weist ein Fahrzeug einen Fahrgastraum, der einen Innenraum mit einer Wand, insbesondere einer Innendecke bildet, und mindestens eine an der Wand angeordnete hierin beschriebene Innenraumluftdesinfektionsvorrichtung auf.

Gemäß noch eines Ausführungsbeispiels weist ein Gebäude einen Innenraum mit einer Wand, insbesondere einer Innendecke, und mindestens eine an der Wand angeordnete hierin beschriebene Innenraumluftdesinfektionsvorrichtung auf.

Es versteht sich, dass die Innenräume auch mit zwei oder noch mehr der hierin beschriebenen Innenraum Luftdesinfektionsvorrichtungen ausgerüstet sein können.

Die vorstehend beschriebenen Ausführungsformen können miteinander kombiniert werden.

Weitere vorteilhafte Ausgestaltungen, Einzelheiten, Aspekte und Merkmale der vorliegenden Erfindung ergeben sich aus den Unteransprüchen, der Beschreibung sowie den beigefügten Zeichnungen. Darin zeigt:
Fig. 1A eine schematische Querschnittsansicht einer Vorrichtung zur Desinfektion von Luft in Innenräumen gemäß einem Ausführungsbeispiel;
Fig. 1B eine schematische Querschnittsansicht einer weiteren Vorrichtung zur Desinfektion von Luft in Innenräumen gemäß einem Ausführungsbeispiel;
Fig. 1C eine schematische Aufsicht auf die in den Figuren 1A und 1B dargestellten Vorrichtungen gemäß einem Ausführungsbeispiel;
Fig. 2A eine vergrößerte perspektivische Ansicht des Luftauslasses der in Figuren 1B und 1C dargestellten Vorrichtung gemäß einem Ausführungsbeispiel;
Fig. 2B eine vergrößerte perspektivische Ansicht des Lufteinlasses der in Figuren 1B und 1C dargestellten Vorrichtung gemäß einem Ausführungsbeispiel;
Fig. 2C eine schematische Seitenansicht eines Lüfters der in Figuren 1B und 1C dargestellten Vorrichtung gemäß einem Ausführungsbeispiel;
Fig. 2D eine schematische Frontansicht des in Fig. 2C gezeigten Lüfters gemäß einem Ausführungsbeispiel;
Fig. 2E eine schematische Rückansicht des in Fig. 2C und Fig. 2D gezeigten Lüfters gemäß einem Ausführungsbeispiel;
Fig. 3A eine perspektivische Ansicht einer Desinfektionszelle der in Figuren 1B gezeigten Vorrichtung zur Desinfektion von Luft in Innenräumen gemäß einem Ausführungsbeispiel;
Fig. 3B eine weitere perspektivische Ansicht der Desinfektionszelle der in Figuren 1B gezeigten Vorrichtung zur Desinfektion von Luft in Innenräumen gemäß einem Ausführungsbeispiel;
Fig. 4A eine schematische Aufsicht von unten auf eine Vorrichtung zur Desinfektion von Luft in Innenräumen gemäß einem Ausführungsbeispiel;
Fig. 4B eine schematische Aufsicht von unten auf die in Fig. 4A gezeigte Vorrichtung zur Desinfektion von Luft in Innenräumen gemäß einem Ausführungsbeispiel;
Fig. 5 eine weitere schematische Aufsicht von unten auf die in Fig. 4A gezeigte Vorrichtung zur Desinfektion von Luft in Innenräumen gemäß einem Ausführungsbeispiel;
Fig. 6 eine perspektivische Ansicht eines Fahrzeugs mit einem Fahrgastraum und Vorrichtung zur Desinfektion von Luft wie sie in den Figuren 1A bis 5 dargestellt sind gemäß einem Ausführungsbeispiel; und
Fig. 7 eine perspektivische Ansicht eines Fahrzeugs mit einem Fahrgastraum und einer Vorrichtung zur Desinfektion von Luft gemäß einem Ausführungsbeispiel.

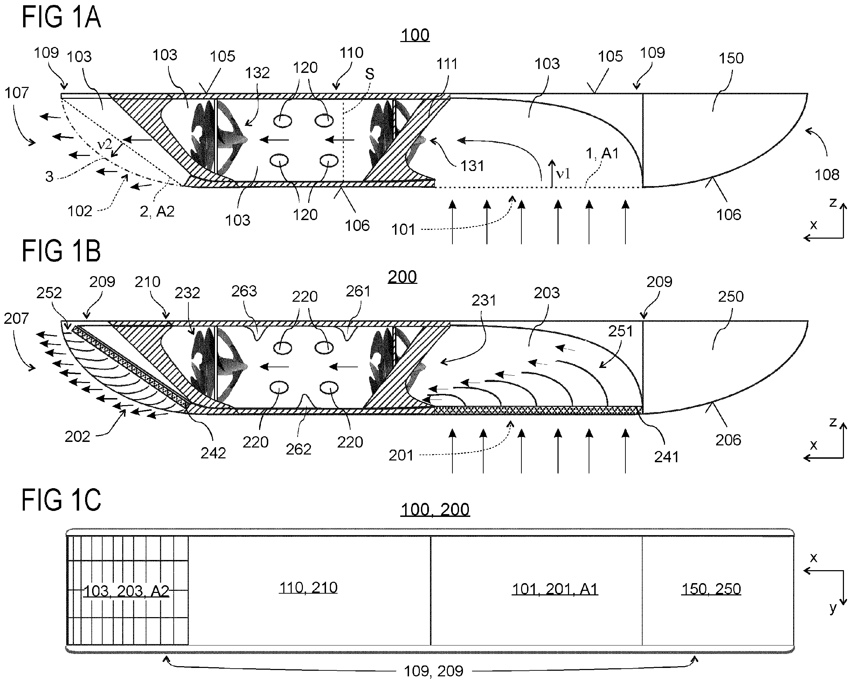

In den Figuren bezeichnen gleiche Bezugszeichen bzw. Bezugszeichen, deren letzte beiden Ziffern übereinstimmen entsprechend ähnliche Teile oder Elemente, die sogar identisch ausgeführt sein können, zumindest dann, wenn dies nicht explizit anders beschrieben wird. Beispielsweise kann ein in Figur 1A mit einem Bezugszeichen 111 versehenes Teil einem gleichen oder ähnlichen Teil in Figur 1B mit einem Bezugszeichen 211 entsprechen.

Zur besseren Orientierung wird in einigen der Figuren zusätzlich ein kartesisches Koordinatensystem (x, y, z) gezeigt, wobei die z-Richtung die vertikale Richtung repräsentiert und die x- bzw. y-Richtung zueinander orthogonal horizontale Richtungen repräsentieren kann.

Außerdem wird die Luftströmung in einigen der Figuren durch Pfeile mit gefüllter Pfeilspitze symbolisiert.

Richtungs- bzw. raumbezogene Begriffe wie "unten", "unter", "unterhalb", "unterer", "über", "oben", "oberhalb" und dergleichen dienen der leichten Beschreibung zur Erklärung der Positionierung eines Elements relativ zu einem zweiten Element. Diese Begriffe sollen verschiedene Ausrichtungen der Vorrichtung zusätzlich zu anderen Ausrichtungen als den in den Figuren dargestellten umfassen.

Fig. 1A zeigt eine schematische Querschnittsansicht bzw. Seitenansicht einer Vorrichtung 100 zur Desinfektion von Luft in Innenräumen.

In dem exemplarischen Ausführungsbeispiel weist die Vorrichtung 100 eine Desinfektionszelle 110 auf, die abgesehen von einer nicht dargestellten optionalen äußeren Verkleidung auf der Stützstruktur 111 der Desinfektionszelle 110 bezüglich einer Längserstreckung (x-Richtung) nach außen einen mittleren Abschnitt eines Gehäuses 109 der Desinfektionszelle 110 bereitstellt, und durch die ein mittlerer Abschnitt eines Strömungskanals 103 führt.

In dem exemplarischen Ausführungsbeispiel ist der Rand des Gehäuses 109 in Querschnitten, die orthogonal zur Längserstreckung (x-Richtung) zumindest abschnittsweise zumindest im Wesentlichen rechtwinklig.

Die Oberseite 105 des Gehäuses 109 ist typischerweise zumindest im Wesentlichen eben, sodass die Vorrichtung 100 an der Oberseite 105 mit einer Innenwand eines Innenraums verbunden werden kann, typischerweise lösbar, zum Beispiel mittels geeigneter, nicht dargestellter Schraubverbindungen.

Dazu sind im Gehäuse 109 oben, insbesondere in der Stützstruktur 109 entsprechende Verankerungsmöglichkeiten und/oder Löcher, zum Beispiel für entsprechende Schrauben vorgesehen.

An einer der Oberseite 105 gegenüberliegenden Unterseite 106 des Gehäuses 109 ist außerhalb der Desinfektionszelle 110 ein gestrichelt dargestellter Lufteinlass 101 zum Strömungskanal 103 gebildet.

Außerdem ist an einer die Oberseite 105 mit der Unterseite 106 verbindende Stirnseite 107 des Gehäuses 109 ein Luftauslass 102 aus dem Strömungskanal 103 gebildet.

Beim Lufteinlass 101 und dem Luftauslass 102 kann es sich jeweils um einfache (zusammenhängende) Öffnungen im Gehäuse 109 handeln (siehe die gestrichelten Linien 1, 2).

Es ist aber auch möglich, dass Lufteinlass 101 und/oder Luftauslass 102 von einer Vielzahl jeweiliger separater Öffnungen gebildet werden. Dies wird in Figur 1A für den Luftauslass 102 durch die Strich-Punkt-Linie 3 dargestellt, die zu dem zeigt, dass der (aerodynamisch effektive) Luftauslass 102 auch eine gekrümmte Fläche sein kann. Dies kann vorteilhaft bezüglich einer besser verteilten Abgabe von Luft in den Innenraum sein.

Wie durch die Pfeile unterhalb des Lufteinlasses 101, im Strömungskanal 103 und links vom Luftauslass 102 dargestellt wird, kann Luft von unten durch den Lufteinlass 101 und damit quer zur Richtung der Längserstreckung des Gehäuses 109 und der mittleren Strömungsrichtung in der Desinfektionszelle 110 (x-Richtung) angesaugt, im Strömungskanal 103 vor der Desinfektionszelle 110 entsprechend umgelenkt, durch den Strömungskanal 103 in der Desinfektionszelle 110 befördert und schließlich durch den Luftauslass 102 zumindest im Wesentlichen in Längserstreckung des Gehäuses (x-Richtung) in den Innenraum abgegeben werden.

Dazu hat die Desinfektionszelle 110 eingangsseitig einen ersten Lüfter 131 zum Ansaugen von Luft und Abgeben der angesaugten Luft in den von der Desinfektionszelle 110 bereitgestellten (mittleren) Strömungskanalabschnitt (im Folgenden auch als mittleren Teil des Strömungskanals 103 und mittleren Strömungskanalabschnitt bezeichnet) und ausgangsseitig einen zweiten Lüfter 132 zum Ansaugen der Luft aus dem Strömungskanalabschnitt der Desinfektionszelle 110 und Abgeben der angesaugten Luft zum Luftauslass 102 hin.

Wie in Figur 1A durch die Linien 1, 2 bzw. 3 dargestellt wird, ist aus Gründen eines effizienten und ruhigen Desinfektionsbetriebs der Vorrichtung für die Luft des Innenraums ein Flächeninhalt A1 des Lufteinlasses 101 (Querschnittsfläche bezüglich der Luftströmung am Lufteinlass 101) kleiner als der Strömungsquerschnitt S im mittleren Strömungskanalabschnitt, und ein Flächeninhalt A2 des Luftauslasses 102 größer als der Strömungsquerschnitt S im mittleren Strömungskanalabschnitt, jedoch kleiner als A1.

Zudem ist typischerweise eine Flächennormale v1 des Lufteinlasses 101 im Wesentlichen senkrecht zum typischerweise zumindest im Wesentlichen rechteckigen Strömungsquerschnitt S im mittleren Strömungskanalabschnitt.

Unter der vorliegend verwendeten Formulierung eines im Wesentlichen rechteckigen Strömungsquerschnitts soll verstanden werden, dass es in Eckereichen und/oder Kantenbereichen Abrundungen geben kann. Zudem kann vorgesehen sein, dass am Rand des Strömungskanals Strömungsumlenkelemente vorgesehen sind.

Der Strömungsquerschnitt im mittleren Strömungskanalabschnitt kann aber auch kreisförmig, elliptisch oder polygonal (typischerweise mit abgerundeten Ecken) ausgeführt sein.

Wie in Figur 1B dargestellt wird, ist der Strömungskanal im mittleren Strömungskanalabschnitt typischerweise zumindest im Wesentlichen zylinderförmigen ausgeführt. Dies bedeutet insbesondere das der Strömungsquerschnitt S im mittleren Strömungskanalabschnitt zumindest im Wesentlichen unabhängig von der Position bezüglich der Strömungsrichtung bzw. Längserstreckung ist.

Es ist aber auch möglich, dass sich der Strömungsquerschnitt S im mittleren Strömungskanalabschnitt zum Luftaustritt 202 hin aufweitet. Dementsprechend kann die Unterseite 106 in diesem Bereich auch geneigt gegenüber der Oberseite 105 und/oder gekrümmt sein.

Außerdem kann eine durch die Flächennormale v1 repräsentierte Orientierung des Lufteinlasses 101 von einer durch die Flächennormale v2 repräsentierte Orientierung des Luftauslasses 102 und der Orientierung des Strömungsquerschnitt S im mittleren Strömungskanalabschnitt abweichen. Im Falle von gekrümmten Ein-/Auslässen 101, 102 gilt dies für mittlere bzw. effektive Flächennormalen.

Zur effektiven Desinfektion von Luft sind im mittleren Strömungskanalabschnitt mehrere UV-Lichtquellen 120 angeordnet.

Damit beim Betrieb der Lichtquellen 120 (Desinfektionsbetrieb der Vorrichtung 100) kein UV-Licht aus der Vorrichtung 100 austreten kann, haben der erste Lüfter 131 und der zweite Lüfter 132 jeweils einen oder mehrere Rotorflügel, jeweils drei in dem exemplarischen Ausgangsbeispiel, die so ausgeführt sind, dass in Strömungsrichtung der Luft im UV-Licht bestrahlten mittleren Strömungskanalabschnitt gesehen kein (direkter) Durchtritt von Licht aus dem mittleren Strömungskanalabschnitt erfolgen kann.

Außerdem weist die Desinfektionszelle 110 eine mechanisch stabile und hinreichend starre Stützstruktur 111, zum Beispiel aus Aluminium zur Befestigung an der Innenwand auf, in der auch die Lüfter 131, 132 befestigt sind.

Somit handelt es sich bei der exemplarischen Desinfektionszelle 110 um eine Sicherheitsdesinfektionszelle.

Außerdem sind die anderen Gehäuseteile, die typischerweise aus einem anderen Material als die Stützstruktur, zum Beispiel einem Kunststoff bestehen, typischerweise mit der Stützstruktur 111 mechanisch verbundenen.

Die Stützstruktur 111 kann einen entsprechenden Rahmen aufweisen bzw. rahmenartig aufgebaut sein und eine oder mehrere typischerweise lösbar mit dem Rahmen verbundene Platten aufweisen.

Insbesondere kann die Stützstruktur 111 an der Unterseite 106 eine entsprechende lösbare Platte oder Klappe aufweisen, die zur Montage der Vorrichtung 100 in der Innenraumwand und/oder für den Wechsel von Komponenten, insbesondere der UV-Lichtquellen 120 abgenommen bzw. geöffnet werden kann.

Durch den gewählten Aufbau der Sicherheitsdesinfektionszelle 110 kann der UV-aktive Bereich zuverlässig gegenüber Vibrationen (die aus dem Fahrbetrieb eines Fahrzeugs herrühren können) aber auch gegenüber Beschädigungen durch Vandalismus zumindest weitgehend geschützt werden.

Typischerweise sind die dargestellten Komponenten direkt oder indirekt mit der Stützstruktur 111 verbunden, die typischerweise zumindest im Wesentlichen, z.B. bis auf etwaige Oberflächenbeschichtung(en) aus einem vibrationsresistenten Material wie Aluminium besteht.

Außerdem kann in/an einer der Stirnseite 107 gegenüberliegenden Stirnseite 108 des Gehäuses 109, die ebenfalls die Oberseite 105 mit der Unterseite 106 verbindendet und typischerweise gekrümmt ausgeführt ist, eine weitere Klappe oder lösbare Platte für die Wandmontage und/oder die Wartung von Komponenten in einem dem Luftauslass 102 gegenüberliegenden Teil 150 der Vorrichtung 100 vorgesehen sein.

In diesem Teil 150 können eine Steuereinheit für andere Komponenten der Vorrichtung 100 wie die Lüfter und UV-Lichtquellen, ein Netzteil und/oder ein elektrischer Energiespeicher zur (temporären) Energieversorgung der UV-Lichtquellen 120 und der Lüfter 131, 132, und/oder der Steuereinheit sowie optional vorhandener Sensoren zur Kontrolle der Betriebsfunktionen und/oder der Komponenten der Vorrichtung 100 untergebracht sein. Außerdem kann der Teil 150 eine elektrische Schnittstelle für die elektrische Energieversorgung, einen An/Ausschalter und/oder eine Schnittstelle zur Datenkommunikation mit der Steuereinheit aufweisen.

Dabei kann die Kapazität des elektrischen Energiespeichers so bemessen sein, dass die Vorrichtung 100 autark (unabhängig von einer stationären elektrischen Versorgung, für den Fall eines Ausfalls der externen Stromversorgung) für 20, 30 oder sogar 45 Minuten betrieben werden kann.

Bei den Sensoren kann sich zum Beispiel und Strömungssensoren für den Strömungskanal 103 sowie UV-Licht-Sensoren, die insbesondere im mittleren Strömungskanalabschnitt angeordnet sein können.

Es kann sich aber auch um Partikelsensoren oder sogar Keimdetektoren handeln, deren an die Steuerung übermittelte Daten zur Steuerung (des Desinfektionsbetriebs) der Vorrichtung 100 verwendet werden können.

Beispielsweise kann vorgesehen sein, die UV Lichtquellen 120 mit erhöhter Leistung und/oder die Lüfter 131, 132 mit etwas geringerer Leistung zu betreiben, wenn die Daten auf eine erhöhte Konzentration von Keimen und/oder auf besonders pathogene Keime deutet.

Zur Erhöhung der Desinfektionseffizienz sind die inneren Oberflächen zumindest im mittleren Strömungskanalabschnitt hoch reflektiv für das verwendete UV-Licht ausgeführt (Reflexionskoeffizienten zumindest 0,9, zumindest 0,95 oder sogar zumindest 0,97).

Alternativ oder zusätzlich kann vorgesehen sein, die inneren Oberflächen photokatalytisch aktiv auszuführen.

Photokatalytisch aktive Oberflächen können sogar für alle von Luft-durchströmten Bereiche und/oder alle Luft-umströmten Komponenten vorgesehen sein.

Fig. 1B zeigt eine schematische Querschnittsansicht bzw. Seitenansicht einer Vorrichtung 200 zur Desinfektion von Luft in Innenräumen. Die Vorrichtung 200 ist ähnlich, in weiten Teilen typischerweise sogar gleich aufgebaut wie die oben mit Bezug zur Figur 1A erläuterte Vorrichtung 100. Dies wird auch durch Figur 1C verdeutlicht, die eine entsprechende Aufsicht auf die Unterseite 106 bzw. 206 der in den Figuren 1A und 1B dargestellten Vorrichtungen 100, 200 zeigt.

Im exemplarischen Ausführungsbeispiel von Figur 1B wird sowohl der Lufteinlass 201 als auch der Luftauslass 202 von einem entsprechenden Schutzgitter 241, 242 gebildet bzw. definiert.

Die Schutzgitter 241, 242 bestehen typischerweise zumindest überwiegend aus einem hinreichend stabilen Material, insbesondere rostfreiem Stahl. Sie stellen damit auch einen guten mechanischen Schutz gegenüber Beschädigungen dar.

Außerdem kann insbesondere das Schutzgitter 241, aber auch das Schutzgitter 242 eine jeweilige Matte aus Stahlwolle aufweisen, die neben dem Filtern grober Partikel auch zu einer gleichmäßigeren Luftströmung und sogar als weiterer UV-Licht Schutz dienen können.

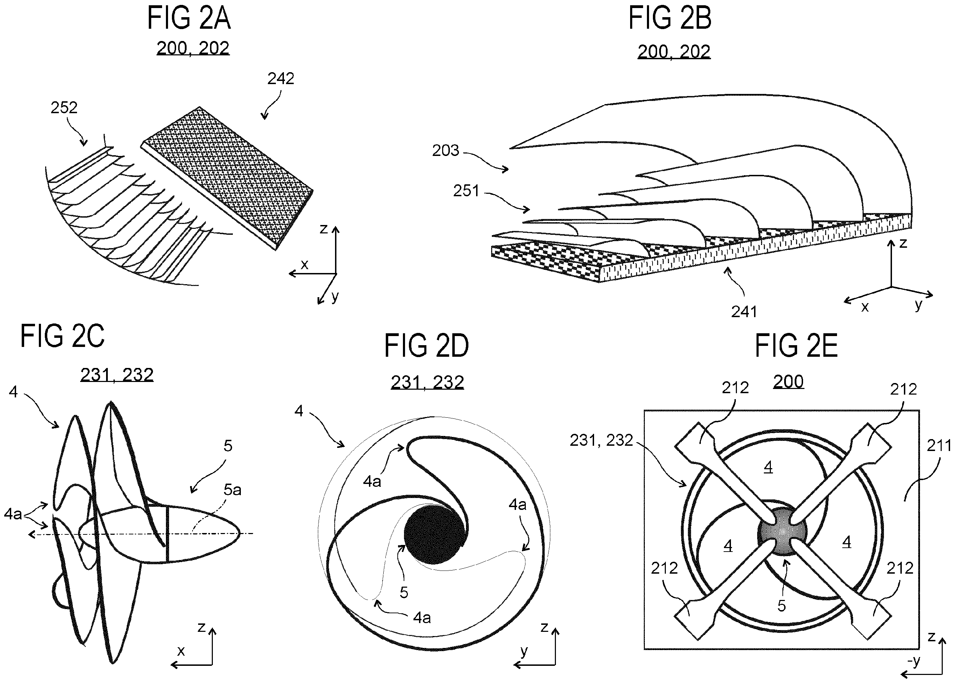

In dem exemplarischen Ausführungsbeispiel sind zudem am Schutzgitter 241 in Strömungsrichtung nachgelagerte gekrümmte und zueinander beabstandete Lamellen 251 angeordnet, zum Beispiel daran befestigt, wie in Figur 2B in einer perspektivischen Darstellung gezeigt ist. Die Lamellen 251 können sowohl dem gleichmäßigeren Ansaugen als auch dem Umlenken der einströmenden Luft zur Desinfektionszelle 210 hin dienen.

Insbesondere können die Lamellen 251 so ausgebildet und angeordnet sein, dass zwischen den Lamellen 251 Strömungsbereiche mit einem jeweiligen Strömungswiderstand gebildet werden, der sich mit zunehmendem Abstand vom ersten Lüfter 231 (negative x-Richtung) verringert.

Außerdem sind auch am Schutzgitter 242 in Strömungsrichtung nachgelagerte, typischerweise weniger stark gekrümmte oder sogar zumindest im Wesentlichen ebene, zueinander beabstandete Lamellen 252 vorgesehen, die in Figur 2A in einer perspektivischen Darstellung detailliert gezeigt sind.

Die Lamellen 252 können bspw. an der Stirnseite 207 des Gehäuses 209 befestigt sein. Durch sie kann eine gleichmäßigere Luftabgabe in den Innenraum und/oder Strömungsaufweitung erleichtert werden.

Mit Bezug zu den Figuren 2C bis 2E wird die Form, Funktion und Aufhängung der in den Figuren 1A, 1B dargestellten Lüfter näher erläutert. Aus Gründen der Übersichtlichkeit werden in den Figuren 2C bis 4E aber nur die Bezugszeichen der Lüfter 231, 232 der Vorrichtung 200 gezeigt. Die Ausführungen gelten aber auch analog für die Lüfter 131, 132 der Vorrichtung 100.

In dem exemplarischen Ausführungsbeispiel weisen die Rotoren 231, 232 jeweils drei mit einem Trägerkörper 5 verbundene speziell (aerodynamisch) geformte Rotorflügel 4 auf, von denen in Figur 2C jedoch nur zwei Rotorflügel 4 mit ihren jeweiligen Spitzen 4a gut erkennbar sind.

Die Rotorflügel 4 ermöglichen einen besonders effizienten und geräuscharmen Pumpbetrieb. Außerdem stellen sie zusammen mit dem Trägerkörper 5 eine effiziente UV-Licht-Barriere dar.

Im exemplarischen Ausführungsbeispiel schirmt jeder der drei Rotorflügel 4 UV-Licht in einem Bereich von 120° bezüglich der Rotorachse 5a um den Trägerkörper 5 ab. Dies ist besonders gut in den Figuren 2D (Ansicht von vorn), 2E (Ansicht von hinten, in Strömungsrichtung Ansaugseite) zu erkennen. Aus Gründen der Übersichtlichkeit ist dabei in Figur 2D einer der drei Rotorflügel 4 als dickere Kurve dargestellt.

Wie bereits oben erläutert wurde, folgen die Rotorflügel 4 (zumindest im Wesentlichen und/oder abschnittsweise) einer jeweiligen Schraubenlinie um die Rotorachse 5a, insbesondere einer räumlichen Kurve, die sich mit variierendem Abstand zur Rotorachse 5a um die Rotorachse 5a windet. Insbesondere kann dabei der Abstand der Schraubenlinie von der Rotorachse 5a ein Maximum aufweisen.

Im Bereich der Rotorspitzen 4a kann von der jeweiligen Schraubenlinie /Kurve auch abgewichen werden (abgeknickte Rotorspitze 4a). Dadurch kann eine gewünschte geräuscharmen Aufweitung der Luftströmung nach dem Passieren der Rotoren 231, 232 erzielt werden.

Im Trägerkörper 5 ist typischerweise ein (Niederspannungsmotor) Elektromotor mit einer typischen Eingangsleistung von 15 W bis 25 W, zum Beispiel 20 W, zum rotatorischen Antrieb der Rotorflügel 4 um die typischerweise parallel zur x-Achse ausgerichtete Rotorachse 5a angeordnet. Die Pumpleistung der Lüfter kann jeweils in einem Bereich von 50-150 m³/h, insbesondere ein Bereich von 60-120 m³/h liegen. Dies kann aber von den Verhältnissen des Innenraums (des pro Zeiteinheit zu desinfizierendes Luftvolumens, geometrische Beschränkungen etc.) sowie der Anzahl der verwendeten Lüfter und/oder der Anzahl der verwendeten Vorrichtungen 100, 200 abhängen.

Wie in Figur 2E dargestellt wird, ist der Trägerkörper 5 typischerweise starr mit der Stützstruktur 211 verbunden, zum Beispiel mittels einer 4-Punkt-Aufhängung über die dargestellten (starren) Verbindungselemente 212.

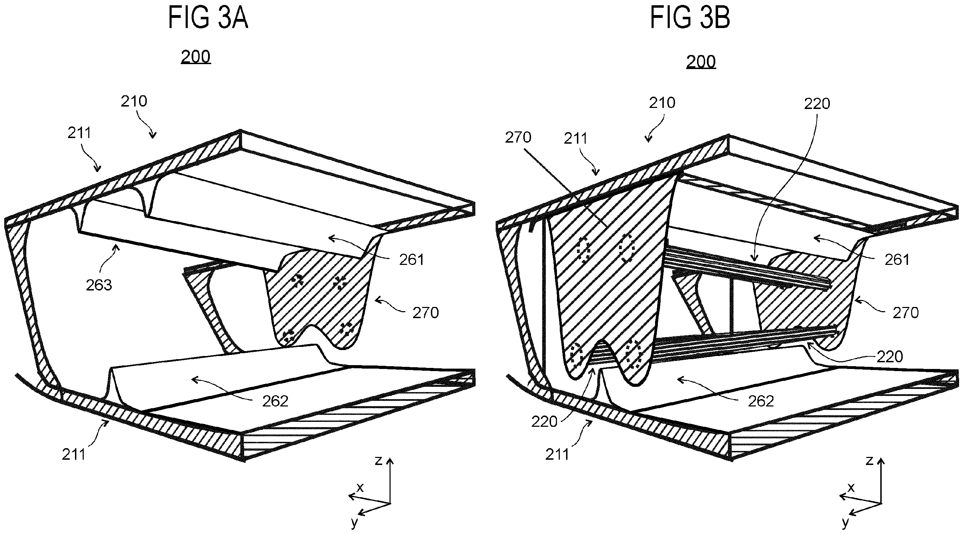

Mit Bezug zu den Figuren 3A und 3B wird ein typischer innerer Aufbau der in Figuren 1B gezeigten Desinfektionszelle 210 mit vier exemplarischen, röhrenförmigen UV-Lichtquellen 120 näher erläutert, die aus Gründen der Übersichtlichkeit in Figur 3A nicht dargestellt und von denen in Figur 3B nur drei deutlich zu erkennen sind. Auch die an einer oberen Deckplatte der Stützstruktur 211 befestigte Trägerstruktur 270 für die UV Lichtquellen 120 ist aus Gründen der besseren Erkennbarkeit der drei Strömungsumlenkelemente 261-263 in Figur 3A nur teilweise (einseitig) gezeigt.

In Ausführungsformen, die sich auf typischerweise leichtere LEDs (LED-Anordnungen) als UV-Lichtquellen 120 beziehen, kann die Trägerstruktur 270 auch anders mit der Stützstruktur 211 verbunden sein, zum Beispiel unten.

Für klassische UV-Niederdruckstrahler aus Quarz als UV-Lichtquellen 120 hat es sich jedoch aus Stabilität- und Schwingungsgründen als vorteilhaft erwiesen, diese über eine Trägerstruktur 270, die typischerweise ebenfalls zumindest im Wesentlichen aus Aluminium oder einem anderen Schwingungsdämpfenden Material besteht und die wie dargestellt oben mit der Stützstruktur 211 zu verbinden. Dies erfolgt zudem typischerweise mittels nicht-dargestellter lösbarer Verbindungelemente (zum Beispiel mittels Schraubverbindungen), um die Wartung und Montage zu erleichtern.

Da die UV-Lichtquellen 120 gut von der Luftströmung umflossen werden können und der Strömungskanalrand typischerweise spiegelnd/hochreflektiv ausgeführt ist, wird eine besonders hohe Desinfektioneffizienz durch die UV-Bestrahlung ermöglicht.

Dazu können auch die aerodynamisch (wellenartig) geformten Strömungsumlenkelemente 261-263 beitragen, die am (rechteckigen) Strömungskanalrand oben bzw. unten und in Richtung der Luftströmung (x-Richtung) in der Nähe der UV-Lichtquellen 120 angeordnet sind und dadurch zu entsprechenden kleinen Verwirbelungen führen können.

Wie besonders gut in Figur 3A erkennbar ist, ist das untere Strömungsumlenkelemente 262 in Strömungsrichtung zwischen den beiden oberen Strömungsumlenkelementen 261, 263 angeordnet. Außerdem sind die Strömungsumlenkelemente 261-263 in Strömungsrichtung versetzt zu den röhrenförmigen und quer zur Strömungsrichtung orientierten UV-Lichtquellen 120 angeordnet.

Beide Maßnahmen tragen dazu bei, dass der exemplarische rechteckige Strömungsquerschnitt nicht zu stark verengt wird.

Zudem sind die typischerweise metallischen Strömungsumlenkelemente 261-263 an ihrer zum Strömungskanal weisenden Oberflächen typischerweise ebenfalls hochreflektiv und/oder photokatalytisch aktiv ausgeführt.

Es versteht sich, dass die beschriebene Aufhängung der UV-Lichtquellen 120 auch für die Vorrichtung 100 verwendet werden kann.

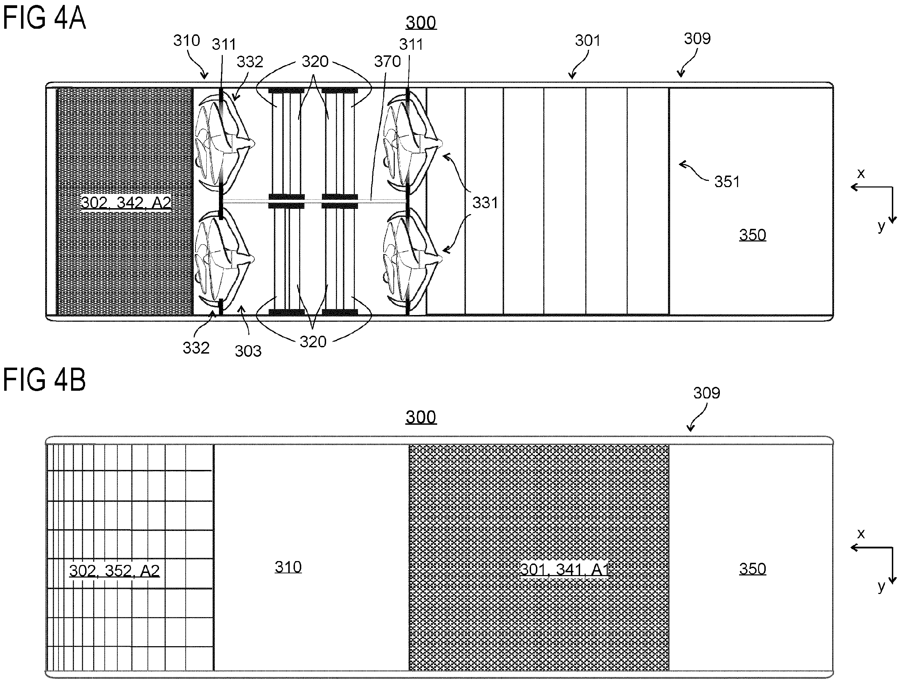

In den Figuren 4A bis 5 wird eine weitere Vorrichtung 300 zur Desinfektion von Luft in Innenräumen dargestellt. Die Vorrichtung 300 ist ähnlich zur oben mit Bezug zu den Figuren 1B bis 3B erläuterte Vorrichtung 200.

Jedoch sind eingangsseitig zwei erste Lüfter 331 und ausgangsseitig zwei zweite Lüfter 332 in Strömungsrichtung (x-Richtung) jeweils parallel nebeneinander angeordnet und mit der Stützstruktur 311 verbunden.

Es hat sich herausgestellt, dass die Verwendung paralleler erster und zweiter Lüfter 331, 332 bei gleicher Pumpleistung - im Vergleich zu entsprechend größeren Lüftern - einen ruhigeren Pumpbetrieb ermöglichen, jedenfalls dann, wenn die hierin beschriebenen Lüfter verwendet werden.

Die Ansicht von unten in Figur 4A entspricht im Wesentlichen der von Figur 4B, wobei das in Figur 4B dargestellt Schutzgitter 341 nicht gezeigt wird, sodass die in Figur 4D verdecken Lamellen 351 am Lufteinlass 301 sichtbar sind, wobei die in Figur 4B dargestellten Lamellen 352 nicht gezeigt werden, sodass das in Figur 4D verdeckte Schutzgitter 342 am Luftauslass 302 sichtbar ist, und wobei in Figur 4B zudem der prinzipielle innere Aufbau der Desinfektionszelle 310 im Strömungskanal erkennbar ist.

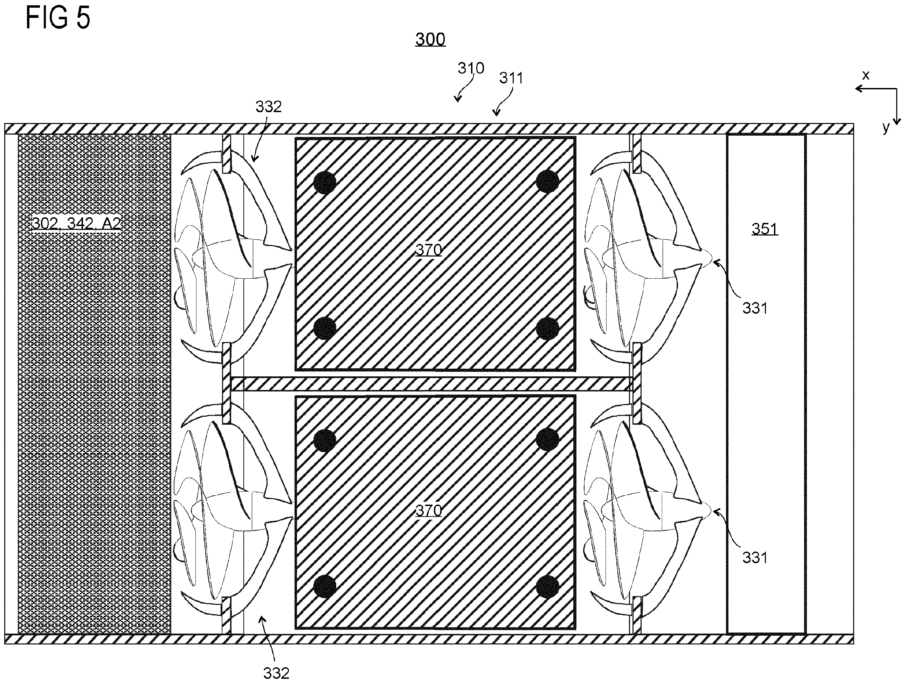

Figur 5 entspricht im Wesentlichen einem vergrößerten Ausschnitt von Figur 4A, jedoch sind anstelle der acht UV-Lichtquellen 120 zwei von der Stützstruktur 311 lösbare rechteckige Platten 370 dargestellt, an denen die UV-Lichtquellen befestigt sind, und die zur Montage bzw. Wartung entfernt werden können.

Es versteht sich, dass auch mehr als zwei, zum Beispiel drei oder vier oder sogar noch mehr erste und zweite Lüfter verwendet werden können.

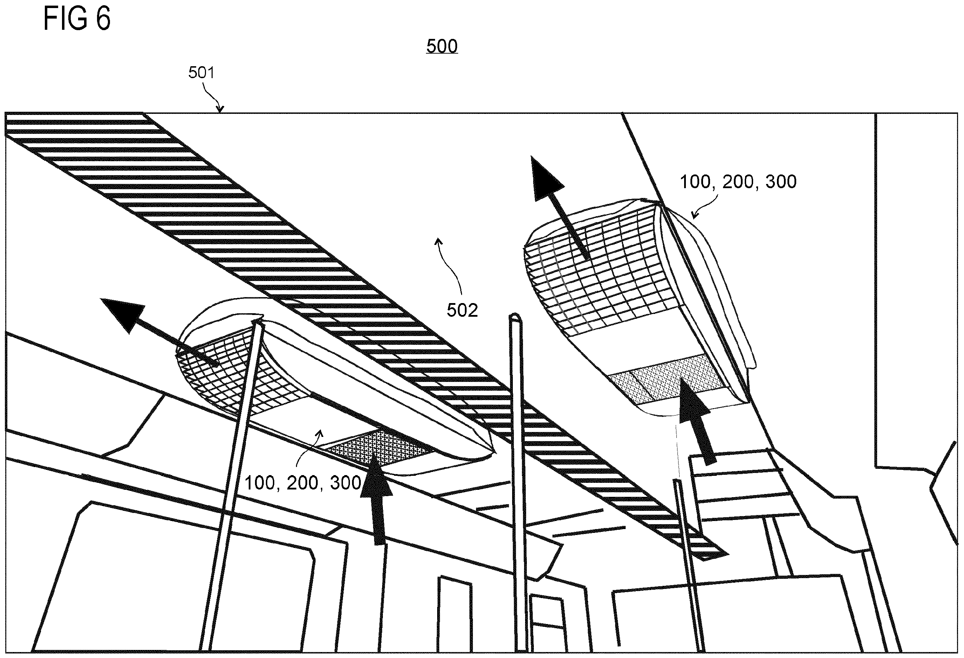

Fig. 6 zeigt eine perspektivische Ansicht eines Fahrzeugs 500 mit einem Fahrgastraum 501 an dessen Decke 502 zwei Luftdesinfektionsvorrichtungen 100, 200, 300 wie sie oben mit Bezug zu den Figuren 1A bis 5 erläutert wurden montiert sind.

Bei dem Fahrzeug 500 handelt es sich typischerweise um ein Schienenfahrzeug insbesondere ein Personenzug für den Nah-, Regional- oder Fernverkehr, eine S-Bahn oder eine U-Bahn.

Es kann sich aber auch um ein Straßenfahrzeug, zum Beispiel ein Bus, ein Wasserfahrzeug oder sogar ein Luftfahrzeug handeln.

Je nach Innenvolumen und geometrischen Verhältnissen des Fahrgastraums kann aber auch nur eine Luftdesinfektionsvorrichtungen 100, 200, 300 oder auch mehr als zwei Luftdesinfektionsvorrichtungen 100, 200, 300 an der Decke 502 montiert sein.

Aufgrund des gewählten Strömungsverlaufs der Vorrichtungen 100-300 kann die Luft aus dem Fahrgastraum 501 effektiv angesaugt und im Wesentlichen parallel zur Decke 502 wieder abgegeben werden.

Dadurch entstehen im Betrieb der Vorrichtungen 100-300 für nicht dargestellte Passagiere keine unangenehmen Luftströmungen.

Außerdem arbeiten die Vorrichtungen 100-300 trotz guter Pumpleistung sehr leise und sind unanfällig gegenüber durch die Fahrt des Fahrzeugs 500 erzeugbaren Schwingungen. Dies reduziert auch den ohnehin geringen Wartungsbedarf weiter.

Zudem ist sowohl der Platzverbrauch als auch der Energieverbrauch der Vorrichtungen 100-300 vergleichsweise gering und die im Übrigen gefällige äußere Form gut in die Fahrzeuggegebenheiten anpassbar.

Neben der Wartung ist auch die Montage der Vorrichtungen 100-300 vergleichsweise einfach, zum Beispiel über Schraubverbindungen realisierbar.

Ein Anschluss an eine gegebenenfalls vorhandene Klimaanlage ist nicht erforderlich. Es wird für einen längeren Betrieb lediglich eine externe elektrische Stromversorgung benötigt.

Somit eignen sich die Vorrichtungen 100-300 auch besonders gut zur Nachrüstung sowohl vorhandener Fahrzeuge als auch von Innenräumen bestehender Gebäude.

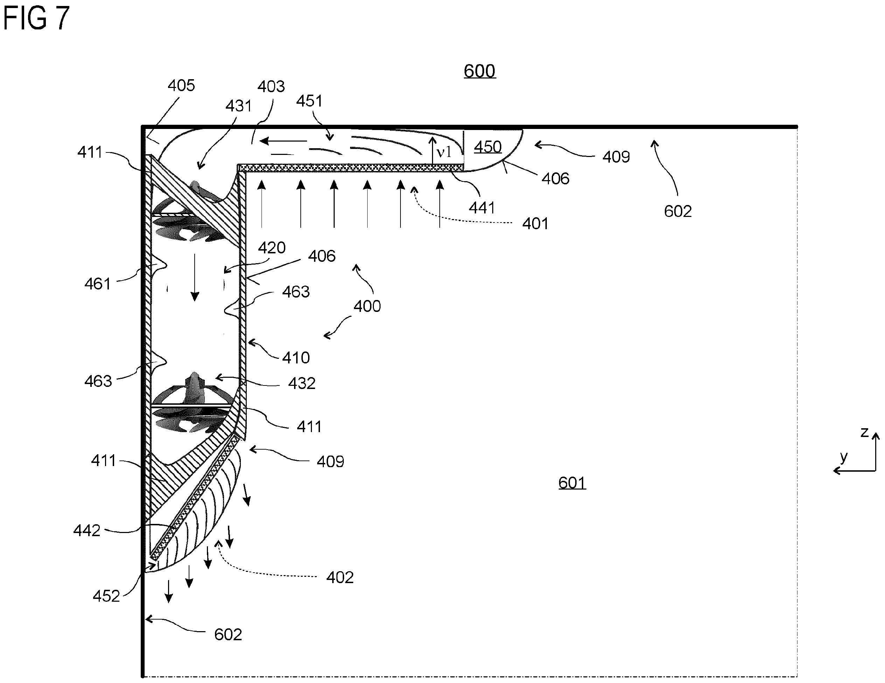

All diese Vorteile gelten zumindest weitgehend auch für die in Fig. 7 dargestellte Innenraumluftdesinfektionsvorrichtung 400, die ähnlich wie die oben mit Bezug zu den Figuren 1A bis 5 beschriebenen Innenraumluftdesinfektionsvorrichtung 100-300 aufgebaut ist, in deren Strömungskanal 403 die Luft jedoch um ca. 180° umgelenkt wird.

Das in Figur 7 dargestellte Ausführungsbeispiel der Innenraumluftdesinfektionsvorrichtung 400 eignet sich besonders für (vergleichbare) flache Innenräume 601 entsprechender Fahrzeuge 600.

Dabei kann vorgesehen sein, die Stützstruktur 411 der Desinfektionszelle 410 mit bzw. an ihrer Oberseite 405 (lösbar) an einer Seitenwand 602 des Innenraums zu befestigen.

Die sich in dieser Ausführungsform um 90° abgeknickte/gedrehte Teile des Gehäuses 409 können (lösbar) mit bzw. an einer Decke 602 des Innenraums 601 befestigt sein/werden.

Zudem ist typischerweise eine Flächennormale v1 des Lufteinlasses 401 im Wesentlichen im 90° Winkel zum typischerweise zumindest im Wesentlichen rechteckigen Strömungsquerschnitt, der in Höhe und Breite im vorderen Strömungskanalabschnitt bis zur Desinfektionszelle 410 auch variieren kann.

Dabei kann vorgesehen sein, den Strömungskanal 403 im vorderen Abschnitt so flach wie nötig gegebenenfalls so flach wie möglichen zu halten.

In dem exemplarischen Ausführungsbeispiel ist das Schutzgitter 441 in einem Winkel von 90° zur mittleren Strömungsrichtung (senkrechter, auf dem Kopf stehender Pfeil) im Strömungskanal der Desinfektionszelle 410 bzw. zum von der Desinfektionszelle 410 gebildeten Abschnitt der Unterseite 406 angeordnet.

Es versteht sich jedoch, dass dieser Winkel entsprechend der Gegebenheiten des Fahrgast- bzw. Innenraums und/oder einer angestrebten Lage/Position der Innenraumluftdesinfektionsvorrichtung 400 darin angepasst sein kann. Dementsprechend kann der Winkel zwischen dem Schutzgitter 441 und der mittleren Strömungsrichtung im Strömungskanal der Desinfektionszelle 410 und/oder dem von der Desinfektionszelle 410 gebildeten Abschnitt der Unterseite 406 auch von 90° abweichen, insbesondere größer als 90° sein, und/oder in einem Bereich von 90° bis 180°, zum Beispiel in einem Bereich von 100° bis 140° liegen, wenn die Innenraumluftdesinfektionsvorrichtung 400 in einem entsprechend gewinkelt oder gekrümmt ausgeführten Übergangsbereichs zwischen einer Seitenwand und einer Deckenwand des Raumes anzuordnen, insbesondere an einer Seitenwand- und Deckenbekleidung des Raumes zu befestigen ist.

Gemäß einem Ausführungsbeispiel weist eine Vorrichtung zur Desinfektion von Luft in Innenräumen von Fahrzeugen zur Personenbeförderung und/oder von Gebäuden einen Lufteinlass mit einer ersten Querschnittsfläche und einen Luftauslass mit einer zweiten Querschnittsfläche, die typischerweise kleiner als die erste Querschnittsfläche ist, auf. Ein Strömungskanal verläuft vom Lufteinlass durch eine Sicherheitsdesinfektionszelle zum Luftauslass. In der Sicherheitsdesinfektionszelle weist der Strömungskanal einen Strömungsquerschnitt auf, der kleiner als die erste Querschnittsfläche und kleiner als die zweite Querschnittsfläche ist. Die Sicherheitsdesinfektionszelle weist zwei hintereinander angeordnete Lüfter zum Erzeugen einer Luftströmung im Strömungskanal vom Lufteinlass zum Luftauslass sowie eine in Richtung der Luftströmung zwischen den zwei Lüftern angeordnete UV-Lichtquelle zum Bestrahlen des Strömungskanals auf. Der erste Lüfter und der zweite Lüfter sind jeweils so ausgeführt ist, dass sie von Licht nicht passiert werden können.

Die vorliegende Erfindung wurde anhand von Ausführungsbeispielen erläutert. Diese Ausführungsbeispiele sollen keinesfalls als einschränkend für die vorliegende Erfindung verstanden werden. Insbesondere können einzelne Merkmale der verschiedenen Ausführungsbeispiele in andere Ausführungsformen übernommen werden oder verschiedene Ausführungsbeispiele miteinander kombiniert werden, solange sich die kombinierten Merkmale nicht technisch bedingt gegenseitig ausschließen.

## Patentansprüche

1. Vorrichtung (100, 200, 300, 400) zur Desinfektion von Luft in Innenräumen, aufweisend:
ein Gehäuse (109, 209, 309, 409) mit einer Längserstreckung und einer Oberseite (105, 205, 305, 405) zum Montieren an einer Innenwand des Innenraums, insbesondere einer Innendecke des Innenraums;
einen Lufteinlass (101, 201, 301, 401) an einer der Oberseite gegenüberliegenden Unterseite des Gehäuses (109, 209, 309, 409), sodass die Luft von unten durch den Lufteinlass (101, 201, 301, 401) quer zur Längserstreckung des Gehäuses angesaugt werden kann, wobei der Lufteinlass (101, 201, 301, 401) eine erste Querschnittsfläche (A1) aufweist;
einen Luftauslass (102, 202, 302, 402) an einer die Oberseite mit der Unterseite verbindende Stirnseite des Gehäuses (109, 209, 309, 409), sodass die Luft durch den Luftauslass (102, 202, 302, 402) im Wesentlichen in Längserstreckung des Gehäuses in den Innenraum abgegeben werden kann, wobei der Luftauslass (102, 202, 302, 402) eine zweite Querschnittsfläche (A2) aufweist, die kleiner als die erste Querschnittsfläche (A1) ist;
einen im Gehäuse (109, 209, 309, 409) vom Lufteinlass (101, 201, 301, 401) zum Luftauslass (102, 202, 302, 402) verlaufenden Strömungskanal (103, 203, 303, 403);
eine im Gehäuse (109, 209, 309, 409) angeordnete und/oder einen Teil des Gehäuses (109, 209, 309, 409) bildende Desinfektionszelle (110, 210, 310, 410), die einen Teil des Strömungskanals (103, 203, 403) bildet und in der der Strömungskanal (103, 203, 303, 403) einen Strömungsquerschnitt (S) aufweist, der kleiner als die zweite Querschnittsfläche (A2) ist, wobei die Desinfektionszelle (110, 210, 310, 410) eingangsseitig mindestens einen ersten Lüfter (131, 231, 331,431) zum Ansaugen von Luft vom Lufteinlass (101, 201, 301, 401) und Abgeben der angesaugten Luft in die Desinfektionszelle (110, 210, 310, 410) und ausgangsseitig mindestens einen zweiten Lüfter (132, 232, 332, 432) zum Ansaugen der Luft aus der Desinfektionszelle (110, 210, 310, 410) und Abgeben der angesaugten Luft zum Luftauslass (102, 202, 302, 402) aufweist, und wobei der erste Lüfter (131, 231, 331, 431) und der zweite Lüfter (132, 232, 332, 432) jeweils mindestens einen oder mehrere Rotorflügel aufweisen, der/die so ausgeführt ist/sind, dass er/sie in Strömungsrichtung der Luft gesehen keinen direkten Durchtritt von Licht gestattet/gestatten; und
mindestens eine in der Desinfektionszelle (110, 210, 310, 410) im Strömungskanal (103, 203, 303, 403) angeordnete UV-Lichtquelle (120, 220, 320, 420) zum Bestrahlen der durch den Strömungskanal (103, 203, 303, 403) strömenden Luft.

2. Vorrichtung (100, 200, 300, 400) nach Anspruch 1, wobei eine Flächennormale (v1) der ersten Querschnittsfläche (A1) im Wesentlichen senkrecht zum Strömungsquerschnitt (S) des Strömungskanals in der Desinfektionszelle (110, 210, 310, 410) angeordnet ist, und/oder wobei eine Orientierung der ersten Querschnittsfläche (A1) des Lufteinlasses (101, 201, 301, 401) von einer Orientierung der zweiten Querschnittsfläche (A2) des Luftauslasses (102, 202, 302, 402) und/oder einer Orientierung des Strömungsquerschnitt (S) des Strömungskanals in der Desinfektionszelle (110, 210, 310, 410) abweicht, und/oder wobei der Lufteinlass (101, 201, 301, 401) durch einen Schutzgitter (241, 341, 441) gebildet wird, dem typischerweise in Strömungsrichtung Lamellen (251, 351, 451) zum Umlenken der einströmenden Luft zur Desinfektionszelle (110, 210, 310, 410) nachgelagert sind, wobei die Lamellen (251, 351, 451) typischerweise am Lufteinlass (101, 201, 301, 401) so ausgeführt sind, dass zwischen den Lamellen (251, 351, 451) Strömungsbereiche mit einem Strömungswiderstand gebildet werden, und Strömungsbereiche, die näher am ersten Lüfter (131, 231, 331, 431) verlaufen, einen höheren Strömungswiderstand aufweisen als Strömungsbereiche, die weiter vom ersten Lüfter (131, 132) verlaufen und/oder die Lamellen (251, 351, 451) in einem Querschnitt entlang des Strömungskanals (103, 203, 303, 403) einem jeweiligen glatten, gekrümmten Kurvenabschnitt folgen, und/oder wobei der Luftauslass (102, 202, 302, 402) durch ein Schutzgitter (242, 342, 442) gebildet wird, dem typischerweise in Strömungsrichtung Lamellen (252, 352, 452) zum Führen der ausströmenden Luft nachgelagert sind.

3. Vorrichtung (100, 200, 300, 400) nach einem der vorhergehenden Ansprüche, wobei die Desinfektionszelle (110, 210, 310, 410) eine Stützstruktur (111, 211, 311, 411) zur Befestigung an einer Innendecke aufweist, und/oder wobei das Gehäuse von der Stützstruktur (111, 211, 311, 411) getragen wird.

4. Vorrichtung (100, 200, 300, 400) nach einem der vorhergehenden Ansprüche, wobei die Desinfektionszelle (110, 210, 310, 410) eine UV-Licht reflektierende innere Oberfläche aufweist, die den Strömungskanal (103, 203, 303, 403) zumindest teilweise begrenzt, und/oder wobei die mindestens eine UV-Lichtquelle (120, 220, 320, 420) vom Rand des Strömungskanals (103, 203, 303, 403) und/oder der UV-Licht reflektierenden inneren Oberfläche beabstandet ist, und/oder wobei mehrere UV-Lichtquelle (120, 220, 320, 420) zum Bestrahlen der durch den Strömungskanal (103, 203, 303, 403) strömenden Luft vorgesehen sind, die auch unterschiedliche Spektraleigenschaften aufweisen können.

5. Vorrichtung (100, 200, 300, 400) nach einem der vorhergehenden Ansprüche, wobei im Wandbereich des Strömungskanals (103, 203, 303, 403) in der Desinfektionszelle (110, 210, 310, 410) Strömungsumlenkelemente (261-263, 461-463) angeordnet sind, die den Strömungskanal (103, 203, 303, 403) in einem Querschnitt entlang des Strömungskanals (103, 203, 303, 403) jeweils wellenförmig, asymmetrisch und/oder einer jeweiligen glatten Kurve folgend begrenzen.

6. Vorrichtung (100, 200, 300, 400) nach Anspruch 5, wobei in dem Querschnitt mindestens ein Strömungsumlenkelement (261, 263, 461, 463) an einem ersten Abschnitt des Wandbereichs, insbesondere einem oberen Abschnitt des Wandbereichs angeordnet ist, und ein weiteres Strömungsumlenkelement (262, 462) an einem zum ersten Abschnitt des Wandbereichs gegenüberliegenden zweiten Abschnitt des Wandbereichs, insbesondere einem unteren Abschnitt des Wandbereichs und in Strömungsrichtung versetzt zu dem mindestens einem Strömungsumlenkelement (261, 263, 461, 463) angeordnet ist.

7. Vorrichtung (100, 200, 300, 400) nach einem der vorhergehenden Ansprüche, wobei die mindestens eine UV-Lichtquelle (120, 220, 320, 420) über eine Trägerstruktur (270, 370) mechanisch mit der Stützstruktur der Desinfektionszelle (110, 210, 310, 410) verbunden ist, wobei die Trägerstruktur (270, 370) und die Stützstruktur (111, 211, 311, 411) typischerweise das gleiche Material, insbesondere Aluminium aufweisen, oder wobei die Trägerstruktur (270, 370) separat zur Desinfektionszelle (110, 210, 310, 410) für eine Befestigung an einer Wand, insbesondere einer Innendecke des Innenraums ausgelegt ist.

8. Vorrichtung (100, 200, 300, 400) nach einem der vorhergehenden Ansprüche, wobei mindestens eine innere Oberfläche der Vorrichtung (100, 200, 300, 400) eine photokatalytische Oberfläche ist, insbesondere eine innere Oberfläche der Desinfektionszelle (110, 210, 310, 410), typischerweise die UV-Licht reflektierende innere Oberfläche der Desinfektionszelle (110, 210, 310, 410).

9. Vorrichtung (100, 200, 300, 400) nach Anspruch 8, weiterhin aufweisend eine von der mindestens eine UV-Lichtquelle (120, 220, 320, 420) separate Lichtquelle zur Bestrahlung der photokatalytischen Oberfläche.

10. Vorrichtung (100, 200, 300, 400) nach einem der vorhergehenden Ansprüche, wobei der erste Lüfter (131, 231, 331, 431, 331) und/oder der zweite Lüfter (132, 232, 332, 431, 332) jeweils eine Rotorachse aufweisen und mindestens einen um die Rotorachse rotierbaren Rotorflügel aufweisen, wobei der mindestens eine Rotorflügel jeden parallel zur Rotorachse auftreffenden Lichtstrahl absorbieren und/oder reflektieren kann, und/oder wobei der mindestens eine Rotorflügel ein Lichtlabyrinth bildet.

11. Vorrichtung (100, 200, 300, 400) nach Anspruch 10, wobei der mindestens eine Rotorflügel zumindest in einem seine Flügelspitze nicht umfassenden Abschnitt im Wesentlichen entlang einer Schraubenlinie um die Rotorachse geformt ist und/oder wobei die Flügelspitze der Schraubenline nicht folgt.

12. Vorrichtung (100, 200, 300, 400) nach einem der vorhergehenden Ansprüche, wobei der erste Lüfter (131, 231, 331, 431) und der zweite Lüfter (132, 232, 332, 432) mit der gleichen Pumpleistung betrieben werden können und/oder zumindest im Wesentlichen gleich aufgebaut sind.

13. Vorrichtung (100, 200, 300, 400) nach einem der vorhergehenden Ansprüche, wobei zwei oder mehrere erste Lüfter (132, 231, 331, 431) und/oder zwei oder mehrere zweite Lüfter (132, 232, 332, 432) in Strömungsrichtung nebeneinander angeordnet sind.

14. Fahrzeug (500, 600), aufweisend einen Fahrgastraum (501, 601), der einen Innenraum mit einer Wand (502, 602, 603), insbesondere einer Innendecke (502, 602) bildet, und mindestens eine an der Wand (502, 602, 603) im Fahrgastraum angeordnete Vorrichtung (100, 200, 300, 400) nach einem der vorhergehenden Ansprüche.

15. Gebäude, aufweisend einen Innenraum mit einer Wand (502, 602, 603), insbesondere einer Innendecke (502, 602), und mindestens eine an der Wand (502, 602, 603) angeordnete Vorrichtung (100, 200, 300, 400) nach einem der Ansprüche 1 bis 13.

## Claims

1. Device (100, 200, 300, 400) for disinfecting air in an interior space, comprising:
a housing (109, 209, 309, 409) having a longitudinal extension and an upper side (105, 205, 305, 405) for installing on an interior wall of the interior space, in particular an interior ceiling of the interior space;
an air inlet (101, 201, 301, 401) at a lower side of the housing (109, 209, 309, 409) opposite the upper side, such that the air can be drawn in, transversely to the longitudinal extension of the housing, through the air inlet (101, 201, 301, 401) from below, the air inlet (101, 201, 301, 401) having a first cross-sectional area (A1);
an air outlet (102, 202, 302, 402) at a front side of the housing (109, 209, 309, 409) connecting the upper side to the lower side, such that the air can be discharged into the interior space substantially in the longitudinal extension of the housing through the air outlet (102, 202, 302, 402), the air outlet (102, 202, 302, 402) having a second cross-sectional area (A2) that is smaller than the first cross-sectional area (A1);
a flow duct (103, 203, 303, 403) extending in the housing (109, 209, 309, 409) from the air inlet (101, 201, 301, 401) to the air outlet (102, 202, 302, 402);
a disinfection cell (110, 210, 310, 410) which is arranged in the housing (109, 209, 309, 409) and/or forms a part of the housing (109, 209, 309, 409), forms a part of the flow duct (103, 203, 403) and in which the flow duct (103, 203, 303, 403) has a flow cross section (S) that is smaller than the second cross-sectional area (A2), wherein the disinfection cell (110, 210, 310, 410) comprises, on an input side, at least one first fan (131, 231, 331, 431) for drawing in air from the air inlet (101, 201, 301, 401) and discharging the drawn-in air into the disinfection cell (110, 210, 310, 410) and, on an output side, at least one second fan (132, 232, 332, 432) for drawing in the air from the disinfection cell (110, 210, 310, 410) and discharging the drawn-in air to the air outlet (102, 202, 302, 402), and wherein the first fan (131, 231, 331, 431) and the second fan (132, 232, 332, 432) each comprise at least one or more rotor blades, which are configured such that they do not allow any light to directly pass through when viewed in the flow direction of the air; and
at least one UV light source (120, 220, 320, 420) arranged in the flow duct (103, 203, 303, 403) in the disinfection cell (110, 210, 310, 410) for irradiating the air flowing through the flow duct (103, 203, 303, 403).

2. The device (100, 200, 300, 400) according to claim 1, wherein a surface normal (v1) of the first cross-sectional area (A1) is arranged substantially perpendicularly to the flow cross section (S) of the flow duct in the disinfection cell (110, 210, 310, 410), and/or wherein an orientation of the first cross-sectional area (A1) of the air inlet (101, 201, 301, 401) differs from an orientation of the second cross-sectional area (A2) of the air outlet (102, 202, 302, 402) and/or an orientation of the flow cross section (S) of the flow duct in the disinfection cell (110, 210, 310, 410), and/or wherein the air inlet (101, 201, 301, 401) is formed by a protective grating (241, 341, 441), downstream of which fins (251, 351, 451) are arranged, typically in the flow direction, for deflecting the inflowing air to the disinfection cell (110, 210, 310, 410), wherein the fins (251, 351, 451) which are typically at the air inlet (101, 201, 301, 401) are configured such that flow regions having a flow resistance are formed between the fins (251, 351, 451), and flow regions that run closer to the first fan (131, 231, 331, 431) have a higher flow resistance than flow regions that run further from the first fan (131, 132) and/or the fins (251, 351, 451) follow a respective smooth, curved section of a curve in a cross section along the flow duct (103, 203, 303, 403), and/or wherein the air outlet (102, 202, 302, 402) is formed by a protective grating (242, 342, 442), downstream of which fins (252, 352, 452) are arranged, typically in the flow direction, for guiding the outflowing air.

3. The device (100, 200, 300, 400) according to any one of the preceding claims, wherein the disinfection cell (110, 210, 310, 410) comprises a supporting structure (111, 211, 311, 411) for fastening to an interior ceiling, and/or wherein the housing is supported by the supporting structure (111, 211, 311, 411).

4. The device (100, 200, 300, 400) according to any one of the preceding claims, wherein the disinfection cell (110, 210, 310, 410) comprises a UV light-reflecting inner surface, which at least partially delimits the flow duct (103, 203, 303, 403), and/or wherein the at least one UV light source (120, 220, 320, 420) is spaced apart from an edge of the flow duct (103, 203, 303, 403) and/or the UV light-reflecting inner surface, and/or wherein a plurality of UV light sources (120, 220, 320, 420) are provided for irradiating the air flowing through the flow duct (103, 203, 303, 403), which UV light sources can also have different spectral properties.

5. The device (100, 200, 300, 400) according to any one of the preceding claims, wherein flow-deflecting elements (261-263, 461-463), which each delimit the flow duct (103, 203, 303, 403) in a cross section along the flow duct (103, 203, 303, 403) in an undulating or asymmetrical manner and/or so as to follow a respective smooth curve, are arranged in a wall region of the flow duct (103, 203, 303, 403) in the disinfection cell (110, 210, 310, 410).

6. The device (100, 200, 300, 400) according to claim 5, wherein in the cross section, at least one flow-deflecting element (261, 263, 461, 463) is arranged at a first portion of the wall region, in particular an upper portion of the wall region, and a further flow-deflecting element (262, 462) is arranged at a second portion of the wall region opposite the first portion of the wall region, in particular a lower portion of the wall region, and so as to be offset from the at least one flow-deflecting element (261, 263, 461, 463) in the flow direction.

7. The device (100, 200, 300, 400) according to any one of the preceding claims, wherein the at least one UV light source (120, 220, 320, 420) is mechanically connected to the supporting structure of the disinfection cell (110, 210, 310, 410) by a carrier structure (270, 370), wherein the carrier structure (270, 370) and the supporting structure (111, 211, 311, 411) typically comprise the same material, in particular aluminum, or wherein the carrier structure (270, 370) is configured separately from the disinfection cell (110, 210, 310, 410) for fastening to a wall, in particular an interior ceiling of the interior space.

8. The device (100, 200, 300, 400) according to any one of the preceding claims, wherein at least one inner surface of the device (100, 200, 300, 400) is a photocatalytic surface, in particular an inner surface of the disinfection cell (110, 210, 310, 410), typically the UV light-reflecting inner surface of the disinfection cell (110, 210, 310, 410).

9. The device (100, 200, 300, 400) according to claim 8, further comprising a light source that is separate from the at least one UV light source (120, 220, 320, 420) for irradiating the photocatalytic surface.

10. The device (100, 200, 300, 400) according to any one of the preceding claims, wherein the first fan (131, 231, 331, 431, 331) and/or the second fan (132, 232, 332, 431, 332) each have a rotor axis and at least one rotor blade rotatable about the rotor axis, wherein the at least one rotor blade can absorb and/or reflect each light beam incident parallel to the rotor shaft, and/or wherein the at least one rotor blade forms a light labyrinth.

11. The device (100, 200, 300, 400) according to claim 10, wherein the at least one rotor blade is shaped substantially along a helix about the rotor axis at least in a portion not including its blade tip, and/or wherein the blade tip does not follow the helix.

12. The device (100, 200, 300, 400) according to any one of the preceding claims, wherein the first fan (131, 231, 331, 431) and the second fan (132, 232, 332, 432) can be operated at the same pumping rate and/or are at least substantially identically in construction.

13. The device (100, 200, 300, 400) according to any one of the preceding claims, wherein two or more first fans (132, 231, 331, 431) and/or two or more second fans (132, 232, 332, 432) are arranged beside one another in the flow direction.

14. A vehicle (500, 600) comprising a passenger compartment (501, 601) which forms an interior having a wall (502, 602, 603), in particular an interior ceiling (502, 602), and at least one device (100, 200, 300, 400) according to any one of the preceding claims arranged on the wall (502, 602, 603) in the passenger compartment.

15. A building comprising an interior having a wall (502, 602, 603), in particular an interior ceiling (502, 602), and at least one device (100, 200, 300, 400) according to any one of claims 1 to 13 arranged on the wall (502, 602, 603).

## Revendications

1. Dispositif (100, 200, 300, 400) pour la désinfection de l'air dans des espaces intérieurs, comprenant :
un boîtier (109, 209, 309, 409) avec une extension longitudinale et un côté supérieur (105, 205, 305, 405) pour le montage sur une paroi interne de l'espace intérieur, plus particulièrement d'un plafond interne de l'espace intérieur ;
une entrée d'air (101, 201, 301, 401) au niveau d'un côté inférieur du boîtier (109, 209, 309, 409), opposé au côté supérieur, de façon à ce que l'air puisse être aspiré à partir du bas à travers l'entrée d'air (101, 201, 301, 401) transversalement par rapport à l'extension longitudinale du boîtier, dans lequel l'entrée d'air (101, 201,3 01, 401) présente une première surface de section transversale (A1) ;
une sortie d'air (102, 202, 302, 402) au niveau d'une face d'extrémité du boîtier (109, 209, 309, 409) reliant le côté supérieur avec le côté inférieur, de façon à ce que l'air puisse être évacuée dans l'espace intérieur à travers la sortie d'air (102, 202, 302, 402) sensiblement dans l'extension longitudinale du boîtier, dans lequel la sortie d'air (102, 202, 302, 402) présente une deuxième surface de section transversale (A2) qui est inférieure à la première surface de section transversale (A1) ;
un canal d'écoulement (103, 203, 303, 403) s'étendant dans le boîtier (109, 209, 309, 409) de l'entrée d'air (101, 201,301, 401) vers la sortie d'air (102, 202, 302, 402) ;
une cellule de désinfection (110, 210, 310, 410) disposée dans le boîtier (109, 209, 309, 409) et/ou formant une partie du boîtier (109, 209, 309, 409), qui forme une partie du canal d'écoulement (103, 203, 303, 403) et dans laquelle le canal d'écoulement (103, 203, 303, 403) présente une section transversale d'écoulement (S) qui est inférieure à la deuxième surface de section transversale (A2), dans lequel la cellule de désinfection (110, 210, 310, 410) comprend, côté entrée, au moins un premier ventilateur (131, 231, 331, 431) pour l'aspiration de l'air par l'entrée d'air (101, 201, 301, 401) et la distribution de l'air aspiré vers la cellule de désinfection (110, 210, 310, 410) et, côté sortie, au moins un deuxième ventilateur (132, 232, 332, 432) pour l'aspiration de l'air provenant de la cellule de désinfection (110, 210, 310, 410) et la distribution de l'air aspiré vers la sortie d'air (102, 202, 302, 402) et dans lequel le premier ventilateur (131, 231, 331, 431) et le deuxième ventilateur (132, 232, 332, 432) comprennent chacun au moins une première ou plusieurs pales de rotor, qui est/sont conçue(s) de façon à qu'il(s) ne permette(nt), vu dans la direction d'écoulement de l'air, aucun passage direct de lumière ; et
au moins une source de lumière UV (120, 220, 320, 420), disposée dans la cellule de désinfection (110, 210, 310, 410) dans le canal d'écoulement (103, 203, 303, 403), pour l'irradiation de l'air s'écoulant à travers le canal d'écoulement (103, 203, 303, 403).

2. Dispositif (100, 200, 300, 400) selon la revendication 1, dans lequel une normale à la surface (v1) de la première surface de section transversale (A1) est disposée sensiblement perpendiculairement à la section transversale d'écoulement (S) du canal d'écoulement dans la cellule de désinfection (110, 210, 310, 410), et/ou dans lequel une orientation de la première surface de section transversale (A1) de l'entrée d'air (101, 201, 301, 401) diffère d'une orientation de la deuxième surface de section transversale (A2) de la sortie d'air (102, 202, 302, 402) et/ou d'une orientation de la section transversale d'écoulement (S) du canal d'écoulement dans la cellule de désinfection (110, 210, 310, 410), et/ou dans lequel l'entrée d'air (101, 201, 301, 401) est constituée d'une grille de protection (241, 341, 441) en aval de laquelle sont disposées habituellement dans la direction d'écoulement des lamelles (251, 351, 451) pour dévier l'air entrant dans la cellule de désinfection (110, 210, 310, 410), dans lequel les lamelles (251, 351, 451) sont conçues sur l'entrée d'air (101, 201, 301, 401) de façon à ce que, entre les lamelles (251, 351, 451), soient formées des zones d'écoulement avec une résistance à l'écoulement, et que des zones d'écoulement qui s'étendent plus près du premier ventilateur (131, 231, 331, 431) présentent une résistance à l'écoulement plus élevée que des zones d'écoulement qui s'étendent plus loin du premier ventilateur (131, 132) et/ou que les lamelles (251, 351, 451) suivent dans une section transversale le long du canal d'écoulement (103, 203, 303, 403) une section courbe lisse respective et/ou dans lequel la sortie d'air (102, 202, 302, 402) est formée par une grille de protection (242, 342, 442) en aval de laquelle sont disposées dans la direction d'écoulement des lamelles (252, 352, 452) pour le guidage de l'air sortant.

3. Dispositif (100, 200, 300, 400) selon l'une des revendications précédentes, dans lequel la cellule de désinfection (110, 210, 310, 410) présente une structure de soutien (111, 211, 311, 411) pour la fixation à un plafond interne et/ou dans lequel le boîtier est supporté par la structure de soutien (111, 211, 311, 411).

4. Dispositif (100, 200, 300, 400) selon l'une des revendications précédentes, dans lequel la cellule de désinfection (110, 210, 310, 410) comprend une surface interne réfléchissant la lumière UV, qui délimite au moins partiellement le canal d'écoulement (103, 203, 303, 403) et/ou dans lequel l'au moins une source de lumière UV (120, 220, 320, 420) est distante du bord du canal d'écoulement (103, 203, 303, 403) et/ou de la surface interne réfléchissant la lumière UV et/ou dans lequel plusieurs sources de lumière UV (120, 220, 320, 420) sont prévues pour l'irradiation de l'air s'écoulant à travers le canal d'écoulement (103, 203, 303, 403), lesquelles peuvent également présenter des propriétés spectrales différentes.

5. Dispositif (100, 200, 300, 400) selon l'une des revendications précédentes, dans lequel, dans la zone de paroi du canal d'écoulement (103, 203, 303, 403) dans la cellule de désinfection (110, 210, 310, 410) sont disposés des éléments de déviation d'écoulement (261 - 263, 461 - 463) qui délimitent le canal d'écoulement (103, 203, 303, 403) dans une section transversale le long du canal d'écoulement (103, 203, 303, 403) respectivement de manière ondulée, asymétrique et/ou en suivant une courbe lisse.

6. Dispositif (100, 200, 300, 400) selon la revendication 5, dans lequel, dans la section transversale, est disposé au moins un élément de déviation d'écoulement (261, 263, 461, 463) au niveau d'une première portion de la zone de paroi, plus particulièrement une portion supérieure de la zone de paroi, et un autre élément de déviation d'écoulement (262, 462) est disposé au niveau d'une deuxième portion de la zone de paroi, opposée à la première portion de la zone de paroi, plus particulièrement une portion inférieure de la zone de paroi, et, dans la direction d'écoulement, décalée par rapport à au moins un élément de déviation d'écoulement (261, 263, 461, 463).

7. Dispositif (100, 200, 300, 400) selon l'une des revendications précédentes, dans lequel l'au moins une source de lumière UV (120, 220, 320, 420) est reliée mécaniquement, par l'intermédiaire d'une structure porteuse (270, 370), avec la structure de soutien de la cellule de désinfection (110, 210, 310, 410), dans lequel la structure porteuse (270, 370) et la structure de soutien (111, 211, 311, 411) présentent un même matériau, plus particulièrement de l'aluminium, ou dans lequel la structure porteuse (270, 370) est réalisée séparément de la cellule de désinfection (110, 210, 310, 410) pour une fixation à une paroi, plus particulièrement à un plafond interne de l'espace intérieur.

8. Dispositif (100, 200, 300, 400) selon l'une des revendications précédentes, dans lequel au moins une surface interne du dispositif (100, 200, 300, 400) est une surface photocatalytique, plus particulièrement une surface interne de la cellule de désinfection (110, 210, 310, 410), la surface interne réfléchissant la lumière UV de la cellule de désinfection (110, 210, 310, 410).

9. Dispositif (100, 200, 300, 400) selon la revendication 8, comprenant en outre une source de lumière séparée de l'au moins une source de lumière UV (120, 220, 320, 420) pour l'irradiation de la surface photocatalytique.

10. Dispositif (100, 200, 300, 400) selon l'une des revendications précédentes, dans lequel le premier ventilateur (131, 230, 331, 431, 331) et/ou le deuxième ventilateur (132, 232, 332, 431, 332) comprennent chacun un axe de rotor et au moins une pale de rotor tournant autour de l'axe de rotor, dans lequel l'au moins une pale de rotor peut absorber et/ou réfléchir chaque rayon de lumière arrivant parallèlement à l'axe de rotor et/ou dans lequel l'au moins une pale de rotor forme un labyrinthe de lumière.

11. Dispositif (100, 200, 300, 400) selon la revendication 10, dans lequel l'au moins une pale de rotor est formée, au moins dans une portion ne comprenant pas son extrémité de pale, sensiblement le long d'une ligne hélicoïdale autour de l'axe de rotor et/ou dans lequel l'extrémité de la pale ne suit pas la ligne hélicoïdale.

12. Dispositif (100, 200, 300, 400) selon l'une des revendications précédentes, dans lequel le premier ventilateur (131, 231, 331, 431) et le deuxième ventilateur (132, 232, 332, 432) peuvent fonctionner avec la même puissance de pompage et/ou sont construits de manière au moins sensiblement identique.

13. Dispositif (100, 200, 300, 400) selon l'une des revendications précédentes, dans lequel deux ou plusieurs premiers ventilateurs (132, 231, 331, 431) et/ou deux ou plusieurs deuxièmes ventilateurs (132, 232, 332, 432) sont disposés les uns à côté des autres dans la direction d'écoulement.

14. Véhicule (500, 600), comprenant un habitacle (501, 601) qui forme un espace intérieur avec une paroi (502, 602, 603), plus particulièrement un plafond interne (502, 602), et au moins un dispositif (100, 200, 300, 400) selon l'une des revendications précédentes, disposé sur la paroi (502, 602, 603) dans l'habitacle.

15. Bâtiment comprenant un espace intérieur avec une paroi (502, 602, 603), plus particulièrement un plafond interne (502, 602), et au moins un dispositif (100, 200, 300, 400) selon l'une des revendications 1 à 13, disposé sur la paroi (502, 602, 603).
